(19) Europäisches Patentamt
European Patent Office
Office européen des brevets

(11) EP 0 876 215 B1

(12) **EUROPÄISCHE PATENTSCHRIFT**

(45) Veröffentlichungstag und Bekanntmachung des Hinweises auf die Patenterteilung:
**04.06.2003 Patentblatt 2003/23**

(21) Anmeldenummer: **96939899.9**

(22) Anmeldetag: **22.11.1996**

(51) Int Cl.$^7$: **B01J 35/00**, B01J 35/10

(86) Internationale Anmeldenummer:
**PCT/EP96/05162**

(87) Internationale Veröffentlichungsnummer:
**WO 97/020630 (12.06.1997 Gazette 1997/25)**

(54) **AMORPHE MIKROPORÖSE MISCHOXIDKATALYSATOREN MIT KONTROLLIERTER OBERFLÄCHENPOLARITÄT FÜR DIE SELEKTIVE HETEROGENE KATALYSE, ADSORPTION UND STOFFTRENNUNG**

AMORPHOUS, MICROPOROUS MIXED OXIDE CATALYSTS WITH CONTROLLED SURFACE POLARITY FOR SELECTIVE HETEROGENEOUS CATALYSIS, ADSORPTION AND MATERIAL SEPARATION

CATALYSEURS D'OXYDES MIXTES AMORPHES MICROPOREUX AVEC POLARITE SUPERFICIELLE CONTROLEE POUR CATALYSE HETEROGENE SELECTIVE, ADSORPTION ET SEPARATION DE MATIERE

(84) Benannte Vertragsstaaten:
**AT BE CH DE DK ES FR GB IE IT LI LU NL SE**

(30) Priorität: **02.12.1995 DE 19545042**

(43) Veröffentlichungstag der Anmeldung:
**11.11.1998 Patentblatt 1998/46**

(73) Patentinhaber: **Studiengesellschaft Kohle mbH**
**45470 Mülheim an der Ruhr (DE)**

(72) Erfinder: **MAIER, Wilhelm, F.**
**D-45470 Mülheim an der Ruhr (DE)**

(74) Vertreter: **von Kreisler, Alek, Dipl.-Chem. et al Patentanwälte,**
**von Kreisler-Selting-Werner,**
**Bahnhofsvorplatz 1 (Deichmannhaus)**
**50667 Köln (DE)**

(56) Entgegenhaltungen:
**DE-A- 2 148 637     DE-A- 2 165 027**
**DE-A- 2 229 015     DE-A- 2 311 822**
**DE-A- 4 419 195**

Bemerkungen:
Die Akte enthält technische Angaben, die nach dem Eingang der Anmeldung eingereicht wurden und die nicht in dieser Patentschrift enthalten sind.

EP 0 876 215 B1

**Beschreibung**

Die Erfindung betrifft neuartige hochporöse Katalysatormaterialien, deren Polarität der inneren Oberflächen gezielt eingestellt werden kann. Die Materialien bestehen aus Mischoxiden, die 0,1-20 Gew.-% chemisch gebundene organische Reste enthalten. Die Aufgabe dieser organischen Reste ist es, die Hydrophobizität der Materialien gezielt zu steuern und damit Umsatz, Standzeit und Selektivität heterogen katalysierter Reaktionen zu verbessern.

Ein bisher ungelöstes Problem ist die Polarität der inneren Oberflächen von heterogenen Katalysatoren, die sich kaum steuern läßt. Diese Oberflächenpolarität beeinflußt die Wechselwirkung vom Katalysator mit den Reaktanden, Produkten und dem Lösungsmittel oder anderer Begleitstoffe. Bei einem guten Katalysator ist diese Wechselwirkung so ausbalanziert, daß Reaktanden und Produkte schnell und effektiv in dem Porensystem des Katalysators diffundieren, wogegen Begleitstoffe und Nebenprodukte weitgehend aus dem Katalysator herausgehalten werden. Zeolithe nehmen normalerweise so gut Wasser auf, daß sie als Trockenmittel breite Verwendung finden. Vor ihrem Einsatz als Katalysatoren müssen sie daher bei Temperaturen von bis zu 500 C gründlichst getrocknet werden. Es gibt aber Ausnahmen. So ist bekannt, daß die Zeolithe mit Silikalitstruktur trotz der hohen inneren Oberfläche nur sehr wenig Wasser sorbieren, was auf eine gewisse Hydrophobizität deutet (E.M. Fianigen, I.M. Bennett, R.W. Grose, J.P. Cohen, R.L. Polton, R. Kirchener, J.V. Smith, *Nature* 272 (1978) 437). Diese Hydrophobizität wird für die besonderen Eigenschaften Ti-haltiger Silikalite (TS-1, TS-2) als selektive Katalysatoren verantwortlich gemacht (T. Tatsumi, K. Asano, K. Yanagisawa in *Studies in Surface Science and Catalysis* 84 (1994) 1861). Während hydrophile Katalysatoren wie mikroporöse Mischoxidgläser oder Y-Zeolithe nicht für die Oxidation mit $H_2O_2$ geeignet sind, sind die für die Oxidation mit $H_2O_2$ geeigneten kristallinen Ti-Silikalite nicht in der Lage tert-Butyl-Hydroperoxid (TBHP) als Oxidationsmittel zu nutzen.

Die Wasseraufnahmekapazität von ZSM Zeolithen hängt linear vom Al-Gehalt ab (D.H. Olsen, W.O. Haag, R.M. Lago, J. Catal. 61 (1980) 390). Diese Hydrophobizität ist der Grund für die Aktivität von Ti-haltigen Silikaliten (TS-1, TS-2) als selektive Oxidationskatalysatoren mit Wasserstoffperoxid als Oxidationsmittel. Im Gegensatz dazu können die vergleichsweise hydrophilen amorphen $TiO_2$-$SiO_2$-Materialien gleicher Zusammensetzung $H_2O_2$ nicht als Oxidationsmittel nutzen (Sheldon, J. Mol Catal. 7 (1980) 107). Mit diesen Materialien kann man t-Butylhydroperoxid als Oxidationsmittel für selektive Oxidationen nutzen, allerdings neigen diese Katalysatoren zur schnellen Desaktivierung (R.A. Sheldon, J.A. Van Doom, J. Catal. 31 (1973)427). Bis jetzt sind Silikalite die einzigen heterogenen Katalysatoren, die $H_2O_2$ als Oxidationsmittel für selektive Oxidationen unter milden Bedingungen nutzen können. Allerdings nimmt mit zunehmendem Ti-Gehalt die Hydrophobizität ab, so daß hier mit zunehmendem Anteil der katalytisch aktiven Zentren ein gegenläufiger Effekt auftritt (Tatsumi et al. Stud. Surf. Sci. Catal. 84 (1994)1861).

Die besondere Bedeutung der Oberflächenpolarität wird auch bei dem Einsatz von Zeolithen in der Adsorptionstechnik deutlich. Durch Dealuminierung können hydrophobe Zeolithe erzeugt werden, die zur adsorptiven Abluftreinigung und Lösungsmittelrückgewinnung eingesetzt werden können (Otten et al. Chem. Ing. Tech. 64 (1992) 915). Allerdings läßt sich bei andern Zeolithen die Wasseraufnahme nicht so vollständig unterdrücken wie bei den Silikatiten (Günzel et al. Chem. Ing. Tech. 61 (1989) 66).

Zusammenfassend kann festgestellt werden, daß nur sehr wenige Zeolithstrukturen durch Dealuminierung hydrophob gemacht werden können und damit eine extrem begrenzte Auswahl von Materialien für heterogene Katalyse und Adsorption in der Gegenwart von Wasser unter milden Bedingungen zur Verfügung stehen. Weiterhin ist die Dealuminierung ein vergleichsweise aufwendiger zusätzlicher Verfahrensschritt und er führt meist zu signifikantem Strukturverlust, der Ausbidlung von Defektstellen und eine signifikanten Erhöhung unerwünschter amorpher Anteile im Zeolithen. Außerdem läßt sich das Ausmaß der Dealuminierung nicht gezielt einstellen und der Effekt einer erfolgten Dealuminierung auf das gewünschte Verfahren muß empirisch ermittelt werden. Ein bekanntes Verfahren zur Änderung der Oberflächenpolarität ist die nachträgliche Modifizierung. So können zugängliche Oberflächenhydroxylgruppen von Katalysatoren durch Silylierung mit bekannten Silylierungsagenzien wie $(CH_3)_3SiCl$ oder $((CH_3)_3Si)_2NH$ (DE 2311822 C2) oder anderen Reagenzien nachträglich modifiziert werden. Diese nachträgliche Modifizierung hat mehrere Nachteile. Die Silyierung in Poren verändert die Porengeometrie und damit mögliche Formselektivitäten, Hydroxylgruppen in Mikroporen < 1 nm können aufgrund der sperrigen Reagenzien nicht oder nur unvollständig modifiziert werden und die so silylierten oder alkylierten Hydroxygruppen sind als $(CH_3)_3Si$-O-Bindung oder R-O-Bindung nur über eine O-Brükke an die Oberfläche gebunden und bleiben damit hydrolyseanfällig. Es besteht daher ein großer Bedarf nach neuen hochporösen Materialien, deren Polarität der Oberfläche unabhängig von der elementaren Zusammensetzung gezielt bei der Herstellung eingestellt werden kann.

Wir haben nun gefunden, daß die Polarität der inneren und äußeren Oberfläche hochporöser. Mischoxide dadurch einstellbar ist, daß Alkyl- oder Aryloxysilane mit nicht hydrolysierbaren R'-Gruppen vom Typ R'-Si(OR)$_3$ mit den anderen Komponenten des Sol-Gel-Prozesses zur Herstellung der porösen Mischoxide copolykondensiert werden. Damit können hydrophobizitätsgesteuerte Katalysatoren In einem Verfahrensschritt hergestellt werden. Die Herstellung rund Zusammensetzung dieser Materialien kann wie folgt abgekürzt werden:

$$x(RO)_4Si + y(R'Si(OR^1)_3) + z(X)_nM + H_2O \rightarrow (R'\text{-}SiO_{1,5})_x(MO_{n/2})_z SiO_2)_y +$$

$$4x\ ROH + 3yR^1OH + nHX$$

wobei R und $R^1$ geeignete Alkyl- oder Arylgruppen sind, die durchaus gleicher Natur sein können, aber nicht müssen, R' ist die nichthydrolysierbare organische Gruppe, die die Hydrophilie des Materials ändert und M ist ein Element der Gruppe Sl, Ti, AL, Mo, Sn, Zn, V, Mn, Fe, Co, Ni, As, Pb, Sb, Bi, Ru, Re, Cr, W, Nb, Hf, La, Ce, Gd, Ga, In, Tl, Ag, Cu, Li, K, Na, Be, Mg, Ca, Sr und Ba, das meist den Träger der katalytischen Aktivität repräsentiert. X sind die Liganden der löslichen Metallverbindungen, vorzugsweise Halogenide, Alkoxide, Oxyalkoxide, Carboxylate Oxalate, Nitrate, Sulfate, Sulfonate, Sulfide, Acetylacetonate, Glykolate oder Aminoalkoxylate. Das Basismaterial in diesem Beispiel ist $SiO_2$, dieses kann durch $Al_2O_3$, $TiO_2$ oder $ZrO_2$ ersetzt werden, was dann zu den folgenden hydrophoben Gläsern führt:

$$(R'\text{-}SiO_{1,5})_x(MO_{n/2})_z(Al_2O_3)_y\ \text{oder}\ (R'\text{-}SiO_{1,5})_x(MO_{n/2})_z(ZrO_2)_y\ \text{oder}$$

$$(R'\text{-}SiO_{1,5})_x(MO_{n/2})_z(TiO_2)_y.$$

Es handelt sich bei dem hier vorgestellten Verfahren um ein speziell optimiertes Polycondensationsverfahren, das darauf ausgerichtet ist, eine echte chemische Mischung der eingesetzten Alkoxide und Salze weitgehend domänenfrei und mit einer hohen Porosität und engen Porenverteilung zu erhalten. Unser Verfahren arbeitet unter milden Bedingungen ohne die Verwendung von Templaten oder anderen Zusätzen. Es kommt ohne den Einsatz von technisch aufwendigen Hydrothermalbedingungen, hohen Druck oder überkritische Reaktionsbedingungen aus und unterscheidet sich grundsätzlich von anderen Herstellungsverfahren für Mischoxide. Ein nachträgliches Modifizieren der Katalysatoren als zweiter Verfahrensschritt wird vermieden und die über drei Sauerstoffbindungen in der Matrix verankerte $CH_3Si$-Gruppe ist thermisch und langzeitstabiler Bestandteil der Feststoffmatrix. Ein besonderes zusätzliches Merkmal der erfindungsgemäßen Materialien ist die Erzeugung der engen Porenverteilung mit Poren < 3 nm, die in Verbindungen mit der steuerbaren Oberflächenpolarität ein besonderes Feintuning der formselektiven und redox-selektiven Eigenschaften des Katalysators verspricht.

Erfindungsgemäß werden hochporöse amorphe Ti-haltige Siliziumdioxide hergestellt, welche die selektive Epoxidierung von Olefinen mit Wasserstoffperoxid vergleichbar gut oder besser katalysiert als die bekannten Zeolithe TS-1 und TS-2. So erhält man unter standardisierten Bedingungen mit einem methylgruppenhaltigen Glas (1% Ti, 25 % Me-Si, 74 % Si) aus Cyclohexen mit $H_2O_2$ 11 % Cyclohexenoxid, während man mit TS-1 nur <2% an Epoxid erhält. Wir haben gefunden, daß mit einem hydrophoben Ti-Glas (3%Ti, 45% Me-Si, 55 % Si) Propen mit TBHP unter gleichen Reaktionsbedingungen die doppelte Ausbeute an Propylenoxid (Selektivität > 97 %) liefert als ein methylgruppenfreies Glas. Im Vergleich dazu liefert TS-1 mit TBHP keinen Umsatz. Während mit Wasserstoffperoxid und einem hydrophoben TiSi-Glas bevorzugt aus Propylen in Methanol Propylenoxid gebildet wird, ist mit einem methylgruppenfreien TiSi-Glas unter vergleichbaren Bedingungen kein Propylenoxid nachweisbar.

Der Einfluß auf sauer katalysierte t-Butyletherbildung aus Isobuten mit n-Hexanol und 1-Naphtol wurde in einer Konkurrenzreaktion untersucht. Als Nebenprodukte galt es die Bildung von Dimeren und Oligomeren des Isobutens zu unterdrücken und eine Selektivität gegenüber einem der beiden Alkohole zu erzielen. Während in methylfreiem Sn-haltigem Si-Glas (3% Sn, 97 % Si) die Bildung von nahezu 25 % an Isobutendimeren, bezogen auf die gebildeten Ether, eintritt, ist bei methylhaltigem Sn-Si-Glas (30 % Me-Si, 3 % Sn, 67 % Si) keine Dimerenbildung im Produkt nachweisbar. Mit methylfreiem Al-haltigem Si-Glas (3% Al, 97 % Sl) überwiegt die Bildung von Isobutendimeren und die Ausbeute liegt bei 67 % Napthol-t-Butylether und 37 % n-Hexyl-t-Butylether. Bei methylhaltigem Al-Si-Glas (10 % Me-Si, 3 % Al, 87 % Si) bilden sich nur 30 % Isobutendimere relativ zur Etherbildung und die Ausbeute an Ethern steigt auf 57 % n-Hexyl-t-Butylether und 43 % Napthol-t-Butylether. Diese Unterdrückung der Dimerenbildung und Änderung der Etherselektivität mit zunehmendem Methylgehalt der Gläser wird auch bei Zr-haltigen Gläsern fortgesetzt.

Die im folgenden aufgeführten Reaktionen wurden unter vergleichbaren Bedingungen bei niedrigem Umsatz durchgeführt. Bei der Ammoxidation von Cyclohexanon mit $H_2O_2$ und Ammoniak erhält man mit methylgruppenhaltigen Ti-Si-Gläsern eine sechsmal höhere Ausbeute an Oxim (27 %) als mit methylgruppenfreien Ti-Si-Gläsern (5%). Aus Toluol bildet sich mit dem methylgruppenhaltigen Glas (1% Ti, 25 % Me-Si, 74 % Si) und $H_2O_2$ In hoher Selektivität das Benzylhydroperoxid, während sich mit dem TS-1 kein Hydroperoxid bildet und mit dem methylfreien Glas die Ausbeute auf 1/10 sinkt.

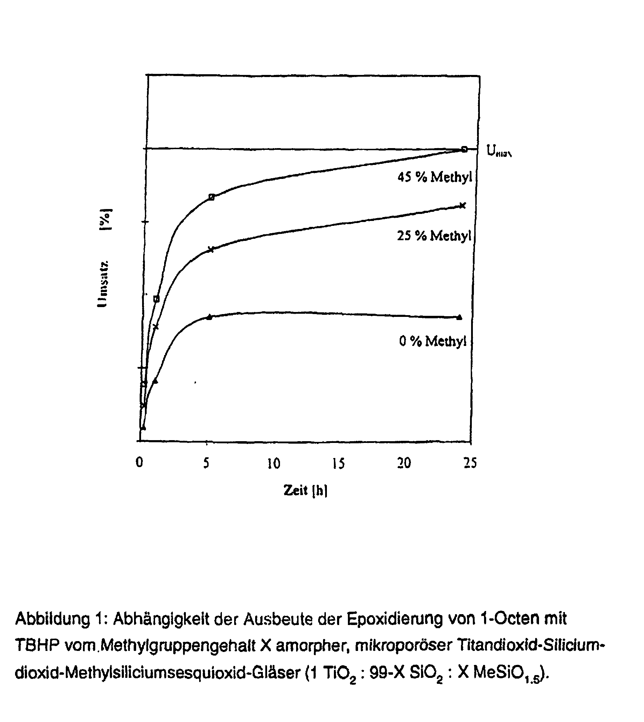

Der aktivitätserhaltende Effekt des kovalenten Einbaues von organischen Gruppen in amorphe poröse Gläser auf die katalytische Aktivität unter standardisierten Bedingungen wird in Abbildung 1 anhand der Epoxidation von 1-OCten mit TBHP deutlich. Wenn 45% aller Si-Atome im Glas kovalent gebundene Methylgruppen enthalten, steigt der Umsatz von maximal 40 % beim methylfreien Glas auf 100 % beim methylhaltigen Glas an. Die frühzeitige Desaktivierung der methylfreien Gläser wird in vielen Reaktionen beobachtet und ist auf Verstopfung der Poren, vermutlich durch Produktmoleküle zurückzuführen, denn diese desaktivierten Katalysatoren lassen sich durch Ausheizen anscheinend beliebig oft und vollständig regenerieren.

Abbildung 1

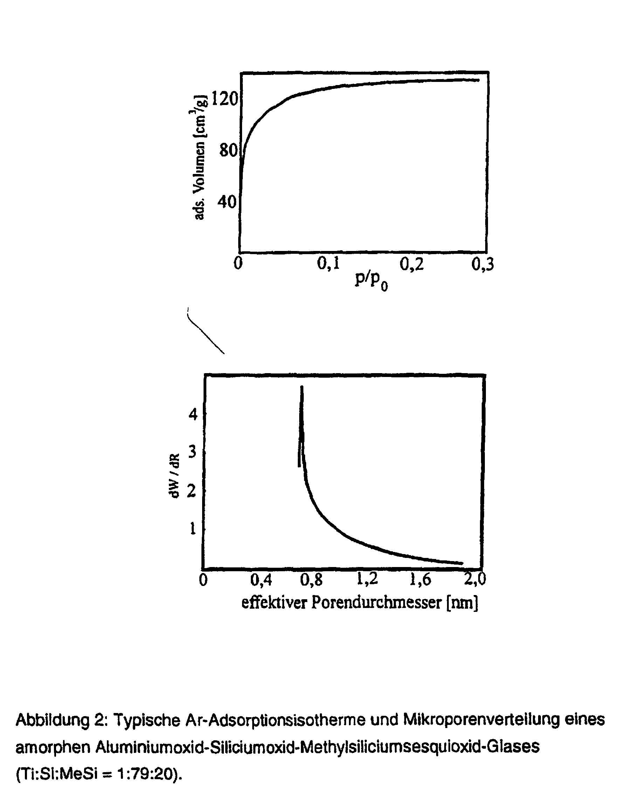

Solche amorphen hydrophoben Gläser können durch das hier beschriebene Sol-Gel-Verfahren in einem Syntheseschritt aus Tetraethoxysilan, Methyltriethoxysilan und Isopropoxytitan hergestellt werden. Von besonderer Bedeutung für diese Erfindung ist, daß die elementaren Bestandteile des fertigen Katalysators homogen ineinander gemischt sind und nicht als Domänen vorliegen. Es handelt sich also um echte chemische Mischoxide. Bei der Herstellung des Sols, in dem alle Komponenten des Endproduktes bereits enthalten sind, ist es wichtig, daß während der Gelierung eine echte Lösung vorliegt und keine Phasentrennung eintritt. Die hier beschriebenen Materialien basieren also alle auf einem Herstellungsprozeß, bei dem mindestens 1-50 mol% $R'-Si(OR)_3$ dem Sol-Gel-Ansatz beigefügt werden. Als nicht hydrolisierbare R'-Gruppen eignen sich grundsätzlich alle chemischen Gruppen, die eine kovalente nichthydrolisierbare stabile Bindung zu Silicium eingehen können. Vorzugsweise werden hier jedoch einfache Gruppen wie Methyl, Ethyl, Isopropyl, $-C_nH_{2n+1}$ und Phenyl- eingesetzt. Es ergibt sich hier aber auch die Möglichkeit, die volle Funktionalität der organischen Chemie zu nutzen, indem als R' funktionalisierte Gruppen wie $-C_nH_{2n}Cl$, $-C_nH_{2n}NH_2$, $-C_nH_{2n}COOH$, $-C_nH_{2n}OH$, $C_nH_{2n}CF_3$, $-CH_2CH=CH_2$, $-CH_2COCH_3$, $-CH_2NR_4^+$ or o, m oder p funktionalisierte Arylgruppen. Diese funktionalisierten Gruppen ermöglichen durch spezifische Wechselwirkung mit bestimmten Substratmolekülen die Diffusion und Adsorption von Reaktanden genau einzustellen. Weiterhin ist die kontrollierte langsame Trocknung der Gele von besonderer Bedeutung für den Erhalt gleichartiger, bekannter enger Porenverteilung. Abbildung 2 zeigt die Adsorptionsisotherme und die Mikroporenverteilung (nach Horvath-Kawazoe) eines methylgruppenhaltigen Al-Si-Glases.

Abbildung 2

Die so erhaltenen Mischoxide unterscheiden sich von allen anderen formselektiven oder hochporösen Mischoxidkatalysatoren dadurch, daß der Katalysator einen definierten Anteil an nichthydrolysierbaren kovalent an Siliciumatome gebundenen organischen Resten enthält. Diese Reste sind maßgeblich für die katalytische Aktivität und Selektivität des Katalysators verantwortlich. Sie unterscheiden sich von den bekannten Materialien (Enichem. EP OS 0492697A1) dadurch, daß es sich bei den jetzt beschriebenen Katalysatoren um Materialien mit der beschriebenen engen Porenverteilung und einer homogenen Elementverteilung handelt und daß unsere Materialien mehr als 1 mol% nicht hydrolisierbare organische Komponenten enthalten.

Das hier vorgestellte Material ist nicht an Mikroporen gebunden. Es können auch größerporige hydrophobe Katalysatormaterialien mit dem Verfahren hergestellt werden.

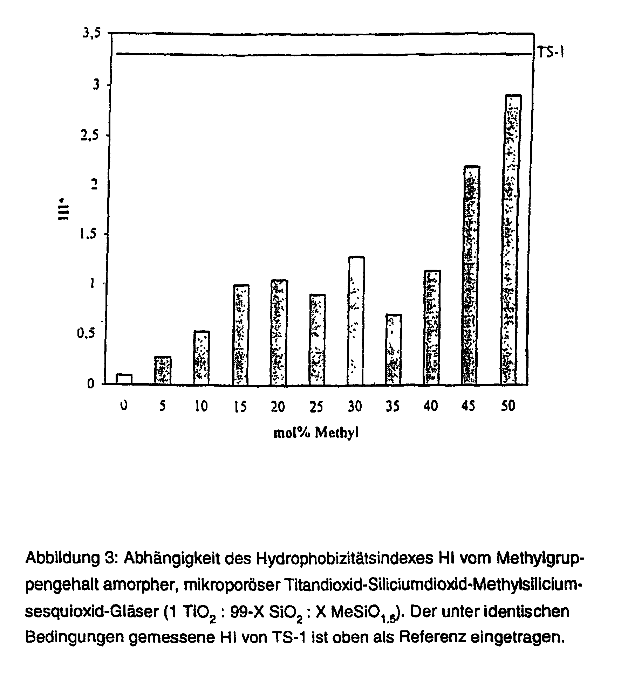

Die Charakterisierung der Oberflächenpolarität der solchermaßen hergestellten Gläser läßt sich nach unterschiedlichen Methoden durchführen. Da unsere Materialien hochporös mit Poren bis in den rein mikroporösen Bereich der kristallinen Zeolithe hergestellt werden können, haben wir für die Charakterisierung ein zur Zeolithcharakterisierung entwickeltes Verfahren eingesetzt (Berke, Kiss, Kleinschmit, Weitkamp, Chem. Ing. Tech. 63 (1991) 623). Hierzu wurde ein mit Wasserdampf und n-Octan gesättigtes Inertgas bei 30 °C über ein Festbett des frisch getrockneten amorphen Mischoxides (standardisierte Kornfraktion und Menge) geleitet und die Durchbruchzeit der Komponenten gaschromatographisch bestimmt. Als quantitatives Maß für die Hydrophobizität kann der Hydrophobizitätsindex HI herangezogen werden, der sich aus dem Verhältnis der Endbeladungen des Materials errechnet. Abbildung 3 zeigt die Abhängigkeit des Hydrophobizitätsindexes von amorphen mikroporösen Siliciumoxiden mit 1 % Titänoxid vom unterschiedlichem Gehalt an nichthydrolisierbaren Methylgruppen. Als Vergleichsstandard dient der in unserem Labor gemessene HI für den extrem hydrophoben TS-1.

Abbildung 3

Mit den hier beschriebenen Materialien kann die Oberflächenpolarität der äußeren und inneren Oberfläche von Mischoxiden so gesteuert werden, daß verschiedene Moleküle allein aufgrund ihrer Polarität an der Diffusion in die Materialien gehindert oder gefördert werden. Das Verfahren ist nicht nur bei mikroporösen, sondern auch bei mesoporösen Materialien, wie z.B. den MCM-Katalysatoren anwendbar, sofern diese aus Alkoxysilanen hergestellt werden, wie in (Mobil Oil, US PS 5,134,242; P.T. Tanev, M, Chibwe, T.J. Pinnavia, Nature 368 (1994) 321) beschrieben. Die hier vorgestellten Materialien können zur selektiven Katalyse von Isomerisierungsreaktionen, Hydrierreaktionen, Oxidationsreaktionen mit Luftsauerstoff, Wasserstoffperoxid oder organischen Peroxiden und Persäuren, Alkylierungs- und Acylierungsreaktionen, Disproportionierungsreaktionen, Hydrier- und Dehydrierreaktionen, Hydratisierungs- und Dehydratisierungsreaktionen, Kondensationsreaktionen, Kupplungsreaktionen, Substitutionsreaktionen, Cycloadditions- und Cycloreversionsreaktionen, Redoxreaktionen, Etherbildung, Ver- und Umesterungsreaktionen, Rohölcracking und Hydrocracking eingesetzt werden. Sie eignen sich für die selektive Katalyse ebenso wie für die Gas- und Flüssigkeitstrennung. Sie können als Ionenaustauscher oder Adsorptionsmittel Verwendung finden. Sie eignen sich auch für die Bildung von Ultrafiltrationsmembranen und Gastrennmembranen und für die Bildung von Katalysatoren mit selektiven Hohlräumen zur molekularen Erkennung (DE-A-4309660). Membranen aus diesen Materialien eignen sich z.B. für die Trennung von Alkohol und Wasser ebenso wie für die Abtrennung von Kohlendioxid aus Luft, Methan aus Grubengas oder Stadtgas, die Reinigung von verbrauchter Luft unter Zurücklassung von Wasser und Kohlendioxid, die Trennung von Kohlenmonoxid und Wasserstoff, Sauerstoff-Stickstofftrennung, die selektive Abtrennung von Wasserstoff aus Gasmischungen, die Rückgewinnung von organischen Lösungsmitteln und die selektive Abtrennung von polaren oder unpolaren Bestandteilen aus Luft, Abluft, Rauchgasen, Küchenluft und verschmutzer Luft aus Klimanlagen.

Beispiel 1

Amorphes, mikroporöses Titandioxid-Siliciumdioxid-Methylsiliciumsesquioxid-Glas (1 $TiO_2$ : 79,2 $SiO_2$ : 19,8 $MeSiO_{1.5}$)

8 ml Tetraethoxysilan (TEOS), 1,8 ml Methyltriethoxysilan (MTES), 0,133 ml Tetraiso-propoxytitan (TIPOT) und 7,9 ml Ethanol werden nacheinander ineinander gelöst und unter Rühren mit 1,98 ml 8N HCl versetzt. Nach erfolgter Erhärtung des Gels wird dieses unter Schutzgas mit einer Aufheizrate von 0,2 C/Min. auf 65 C aufgeheizt, 3h bei dieser Temperatur gehalten, mit einer Aufheizrate von 0,2 C/Min. auf 250 C gebracht und weitere 3h bei dieser Temperatur kalziniert. Adsorptions-Desorptionsisothermen zeigen, daß das Material eine monomodale Mikroporenverteilung aufweist, $S_{BET}$ = 545 $m^2$/g, Porendurchmesser: 0,72 nm.

Beispiel 2

Amorphe, mikroporöse Titandioxid-Siliciumdioxid-Methylsiliciumsesquioxid-Gläser der Zusammensetzung 1 $TiO_2$ : 50,95 - 100 $SiO_2$ : 0 - 49,5 $MeSiO_{1.5}$)

Analog Beispiel 1 wurden Titandioxid-Siliciumdioxid-Methylsiliciumsesquioxid-Gläser aus 10 ml TEOS (BET: 585, Porendurchmesser 0,73 nm), 9,5 ml TEOS und 0,45 ml MTES (BET: 489 $m^2$/g, Porendurchmesser 0,74 nm), 9 ml TEOS und 0,9 ml MTES (BET: 627 $m^2$/g, Poren-durchmesser 0,73 nm), 8,5 ml TEOS und 1,35 ml MTES (BET: 527 $m^2$/g, Porendurchmesser 0,73 nm), 8,0 ml TEOS und 1,8 ml MTES (BET: 545 $m^2$/g, Porendurchmesser 0,72 nm) 7,5 ml TEOS und 2,23 ml MTES (BET: 532 $m^2$/g, Porendurchmesser 0,73 nm), 7,0 ml TEOS und 2,76 ml MTES (BET: 639 $m^2$/g, Porendurchmesser 0,74 nm), 6,5 ml TEOS und 3,15 ml MTES (BET: 575 $m^2$/g, Porendurchmesser 0,73 nm), 6,0 ml TEOS und 3,6 ml MTES (BET: 625 $m^2$/g, Porendurchmesser 0,73 nm), 5,5 ml TEOS und 4,05 ml MTES (BET: 574 $m^2$/g, Porendurchmesser 0,72 nm) sowie 5 ml TEOS und 4,5 ml MTES (BET: 580 $m^2$/g, Porendurch-messer 0,73 nm) bei gegenüber Beispiel 1 unveränderter Vorgehensweise und unveränderten Mengen an TIPOT (0,133 ml), Ethanol (7,9 ml) und 8N HCl (1,98 ml) hergestellt.

Beispiel 3

Amorphes, mikroporöses Titandioxid-Siliciumdioxid-Phenylsiliciumsesquioxid-Glas

Analog Beispiel 1 wurde aus 6,5 ml TEOS, 3,78 ml Phenyltriethoxysilan, 0,133 ml TIPOT, 7,9 ml Ethanol durch Zugabe von 1,98 ml 8N HCl ein Gel der Zusammensetzung 1 $TiO_2$: 64,35 $SiO_2$: 34,65 $(C_6H_5)SiO_{1.5}$ gewonnen, das dem unter Beispiel 1 beschriebenen Kalzinierungsprozeß unterworfen wurde.

Beispiel 4

Amorphes, mikroporöses Tltandioxid-Siliciumdioxid-Ethylsiliciumsesquioxid-Glas

Analog Beispiel 1 wurde aus 9 ml TEOS, 0,96 ml Ethyltriethoxysilan, 0.133 ml TIPOT, 7,9 ml Ethanol durch Zugabe von 1,98 ml 8N HCl ein Gel der Zusammensetzung 1 $TiO_2$ : 89 $SiO_2$ : 10 $(C_2H_5)SiO_{1.5}$ gewonnen, das dem unter Beispiel 1 beschriebenen Kalzinierungsprozeß unterworfen wurde.

Beispiel 5

Amorphes, mikroporöses Titandioxid-Siliciumdioxid-n-propyisiliciumsesquioxid-Glas

Analog Beispiel 1 wurde aus 9 ml TEOS, 0,79 ml n-propyltrimethoxysilan, 0,133 ml TIPOT, 7,9 ml Ethanol durch Zugabe von 1,98 ml 8N HCl ein Gel der Zusammensetzung 1 $TiO_2$ : 89 $SiO_2$ : 10 $(C_3H_7)SiO_{1.5}$ gewonnen, das dem unter Beispiel 1 beschriebenen Kalzinierungsprozeß unterworfen wurde.

Beispiel 6

Amorphes, mikroporöses Titandioxid-Siliciumdioxid-Isobutylsiliciumsesquioxid-Glas

Analog Beispiel 1 wurde aus 9 ml TEOS, 1,12 ml Isobutyltriethoxysilan, 0,133 ml TIPOT, 7,9 ml Ethanol durch Zugabe von 1,98 ml 8N HCl ein Gel der Zusammensetzung 1 $TiO_2$ : 89 $SiO_2$ :10 $(C_4H_9)SiO_{1.5}$ gewonnen, das dem unter Beispiel 1 beschriebenen Kalzinierungsprozeß unterworfen wurde.

Beispiel 7

Amorphes, mikroporöses Titandioxid-Siliciumdioxid-n-Hexyisiliciumsesquioxid-Glas

Analog Beispiel 1 wurde aus 6,5 ml TEOS, 3,60 ml n-Hexyltrimethoxysilan, 0,133 ml TIPOT, 7,9 ml Ethanol durch Zugabe von 1,98 ml 8N HCl ein Gel der Zusammensetzung 1 $TiO_2$ : 64,35 $SiO_2$ : 34,65 $(n-C_6H_{13})SiO_{1.5}$ gewonnen gewonnen, das dem unter Beispiel 1 beschriebenen Kalzinierungsprozeß unterworfen wurde.

Beispiel 8

Amorphe, mikroporöse Aluminiumoxid-Siliciumdioxid-Methylsiliciumsesquioxid-Gläser

In Anlehnung an Beispiel 1 wurden Aluminiumoxid-Siliciumdioxid-Methylsiliciumsesquioxid-Gläser der Zusammensetzung 1,5 $Al_2O_3$ : 97 $SiO_2$ : 0 $MeSiO_{1.5}$ (0,176 ml Tris(sec.)butoxy-Aluminium, 5 ml TEOS), 1,5 $Al_2O_3$ : 92,15 $SiO_2$ : 4,85 $MeSiO_{1.5}$ (0,176 ml Tris(sec.)-butoxy-Aluminium, 4,75 ml TEOS, 0,22 ml MTES) sowie 1,5 $Al_2O_3$ : 87,3 $SiO_2$ : 9,7 $MeSiO_{1.5}$ (0,176 ml Tris(sec.)butoxy-Aluminium, 4,5 ml TEOS, 0,44 ml MTES) unter Verwendung von jeweils 4,2 ml Ethanol und 1 ml 8N HCl dargestellt. Die Vorgehensweise der Geldarstellung und -kalzinierung ist Beispiel 1 zu entnehmen.

Beispiel 9

Amorphe, mikroporöse Zirkonoxid-Siliciumdioxid-Methylsiliciumsesquioxid-Gläser

In Anlehnung an Beispiel 1 wurden Zirkonoxid-Siliciumdioxid-Methylsiliciumsesquioxid-Gläser der Zusammensetzung 3 $ZrO_2$ : 97 $SiO_2$ : 0 $MeSiO_{1.5}$ (0,311 ml Tetra-n-propoxy-Zirkon (70%ig in n-Propanol), 5 ml TEOS), 3 $ZrO_2$ : 92,15 $SiO_2$ : 4,85 $MeSiO_{1.5}$ (0,311 ml Tetra-n-propoxy-Zirkon (70%ig in n-Propanol), 4,75 ml TEOS, 0,22 ml MTES) sowie 3 $ZrO_2$ : 87,3 $SiO_2$ : 9,7 $MeSiO_{1.5}$ (0,311 ml Tetra-n-propoxy-Zirkon (70%ig in n-Propanol), 4.5 ml TEOS, 0,44 ml MTES) unter Verwendung von jeweils 4,2 ml Ethanol und 1 ml 8N HCl dargestellt. Die Vorgehensweise der Geldarstellung und -kalzinierung ist Beispiel 1 zu entnehmen.

Beispiel 10

Amorphe, mikroporöse Zinnoxid-Siliciumdioxid-Methylslllclumsesquioxid-Gläser

In Anlehnung an Beispiel 1 wurden Zinnoxid-Siliciumdioxid-Methylslllciumsesquioxid-Gläser der Zusammensetzung 3 $SnO_2$ : 97 $SiO_2$ : 0 $MeSiO_{1.5}$ (0,435 ml Zinn (ll)-ethylhexanoat, 10 ml TEOS), 3 $SnO_2$ : 87,3 $SiO_2$ : 9,7 $MeSiO_{1.5}$ (0,435 ml Zinn (II)-ethylhexanoat, 9 ml TEOS, 0,9 ml MTES), 3 $SnO_2$ : 77,6 $SiO_2$ : 19,4 $MeSiO_{1.5}$ (0,435 ml Zinn (ll)-ethylhexanoat, 8 ml TEOS, 1,8 ml MTES) sowie 3 $SnO_2$ : 67,9 $SiO_2$ : 29,1 $MeSiO_{1.5}$ (0,435 ml Zinn (ll)-ethylhexanoat, 7 ml TEOS, 2,7 ml MTES) unter Verwendung von jeweils 4,2 ml Ethanol und 1 ml 8N HCl dargestellt. Die Vorgehensweise der Geldarstellung und -kalzinierung ist Beispiel 1 zu entnehmen.

Beispiel 11

Amorphe, mikroporöse Vanadiumoxid-Siliciumdioxid-Methylsiliciumsesquioxid-Gläser

In Anlehnung an Beispiel 1 wurden Vanadiumoxid-Siliciumdioxid-Methylsiliciumsesquioxid-Gläser der Zusammensetzung 2,5 $V_2O_5$ : 95 $SiO_2$ : 0 $MeSiO_{1,5}$ (0,66 g Vanadylacetylacetonat, 11 ml TEOS), 2,5 $V_2O_5$ : 85,5 $SiO_2$ : 9,5 $MeSiO_{1,5}$ (0,66 g Vanadylacetylacetonat, 10 ml TEOS, 1 ml MTES), 2,5 $V_2O_5$ : 76 $SiO_2$ : 19 $MeSiO_{1,5}$ (0,66 g Vanadylacetylacetonat, 9 ml TEOS, 2 ml MTES) sowie 2,5 $V_2O_5$ : 66,5 $SiO_2$ : 28,5 $MeSiO_{1,5}$ (0,66 g Vanadylacetylacetonat, 7,8 ml TEOS, 3 ml MTES) unter Verwendung von jeweils 9 ml Ethanol und 2,25 ml 8N HCl dargestellt. Die Vorgehensweise der Geldarstellung und -kalzinierung ist Beispiel 1 zu entnehmen.

Beispiel 12

Amorphe, mikroporöse Manganoxid-Siliciumdioxid-Methylsiliciumsesquioxid-Gläser

In Anlehnung an Beispiel 1 wurden Manganoxid-Siliciumdioxid-Methylsiliciumsesquioxid-Gläser der Zusammensetzung 3% Mn, 97% Si: 0 MeSi (1,344 mmol Mn(II)acetat, 44,8 mmol TEOS), 3% Mn, 87% Si : 10% MeSi (1,34 mmol Mn(III)acetat, 40,3 mmol TEOS, 4,5 mmol MTES),
3% Mn, 77% Si: 20% MeSi (1,34 mmol Mn(III)acetat, 35,9 mmol TEOS, 9 mmol MTES),
3% Mn, 67% Si:30% MeSi (1,34 mmol Mn(III)acetat, 31,4 mmol TEOS, 13,5 mmol MTES),
3% Mn, 57% Si: 40% MeSi (1,34 mmol Mn(III)acetat, 26,9 mmol TEOS, 17,9 mmol MTES),
3% Mn, 48,5% Si : 48,5% MeSi (1,34 mmol Mn(III)acetat, 22,4 mmol TEOS, 22,4 mmol MTES) unter Verwendung von jeweils 8,1 ml Ethanol und 2 ml 8N HCl dargestellt. Die Komponenten wurden in der reihenfolge TEOS, MTES, Mn(III) acetat*$2H_2O$ und Ethanol in einem 50 ml-PP-Becher gegeben. Unter Rühren wurde die Salzsäure tropfenweise zugegeben. nach 5 min Rührzeit wurde der Becher locker abgedeckt bis zur vollständigen Gelierung. Die Vorgehensweise der Geldarstellung und -kalzinierung ist Beispiel 1 zu entnehmen.

Beispiel 13

Amorphe, mikroporöse Chromoxid-Siliciumdioxid-Methylsiliciumsesquioxid-Gläser

In Anlehnung an Beispiel 1 wurden Chromoxid-Siliciumdioxid-Methylsiliciumsesquioxid-Gläser der Zusammensetzung 3% Cr, 97% Si : 0 MeSi (1,344 mmol Cr(O-iPr)$_3$, 44,8 mmol TEOS), BET: 468 $m^2$/g; 3% Cr, 87% Si : 10% MeSi (1,34 mmol Cr(O-iPr)$_3$, 40,3 mmol TEOS, 4,5 mmol MTES), 571 $m^2$/g; 3% Cr, 77% Si : 20% MeSi (1,34 mmol Cr(O-iPr)$_3$, 35,9 mmol TEOS, 9 mmol MTES),
3% Cr, 67% Si : 30% MeSi (1,34 mmol Cr(O-iPr)$_3$, 31,4 mmol TEOS, 13,5 mmol MTES),
3% Cr, 57% Si : 40% MeSi (1,34 mmol Cr(O-iPr)$_3$, 26,9 mmol TEOS, 17,9 mmol MTES),
3% Cr, 48,5% Si : 48,5% MeSi (1,34 mmol Cr(O-iPr)$_3$, 22,4 mmol TEOS, 22,4 mmol MTES) unter Verwendung von jeweils 8,1 ml Ethanol und 2 ml 8N HCl dargestellt. Die Komponenten wurden in der Reihenfolge TEOS, MTES, Cr (OiPr)$_3$ und Ethanol in einem 50 ml-PP-Becher gegeben. Unter Rühren wurde die Salzsäure tropfenweise zugegeben. nach 5 min Rührzelt wurde der Becher locker abgedeckt bis zur vollständigen Gelierung. Die Vorgehensweise der Geldarstellung und -kalzinierung ist Beispiel 1 zu entnehmen.

Beispiel 14

Amorphe, mikroporöse Eisenoxid-Siliciumdioxid-Methylsiliciumsesquioxid-Gläser

In Anlehnung an Beispiel 1 wurden Eisenoxid-Siliciumdioxid-Methylsiliciumsesquioxid-Gläser der Zusammensetzung 3% Fe, 97% Si : 0 MeSi (1,344 mmol Fe(O-iPr)$_3$, 44,8 mmol TEOS), 3% Fe, 87% Si : 10% MeSi (1,34 mmol Fe(O-iPr)$_3$, 40,3 mmol TEOS, 4,5 mmol MTES), 3% Fe, 77% Si: 20% MeSi (1,34 mmol Fe(O-iPr)$_3$, 35,9 mmol TEOS, 9 mmol MTES), 3% Fe, 67% Si: 30% MeSi (1,34 mmol Fe(O-iPr)$_3$, 31,4 mmol TEOS, 13,5 mmol MTES), 3% Fe, 57% Si : 40% MeSi (1,34 mmol Fe(O-iPr)$_3$, 26,9 mmol TEOS, 17,9 mmol MTES), 3% Fe, 48,5% Si : 48,5% MeSi (1,34 mmol Fe(O-iPr)$_3$, 22,4 mmol TEOS, 22,4 mmol MTES) unter Verwendung von jeweils 8,1 ml Ethanol und 2 ml 8N HCl dargestellt. Die Komponenten wurden in der Reihenfolge TEOS, MTES, Fe(O-iPr)$_3$ und Ethanol In einem 50 ml-PP-Becher gegeben. Unter Rühren wurde die Salzsäure tropfenweise zugegeben. nach 5 min Rührzeit wurde der Becher locker abgedeckt bis zur vollständigen Gelierung. Die Vorgehensweise der Geldarstellung und -kalzinierung ist Beispiel 1 zu entnehmen.

Beispiel 15

Amorphes, mikroporöses Antimonoxid-Zirkondioxid-Titandioxid-Methylsiliciumsesquioxid-Glas

10.00 ml Ti ( OPr$^i$ )$_4$, 1,54 ml Zr (OBu$^n$ )$_4$, 0,63 g Sb(OBu)$_3$, ??ml MeSi(OEt)$_3$, 10,65 ml n-werden nacheinander ineinander unter Ar gelöst. Die klare Lösung wird für 15 min gerührt. Nach Zugabe von 10,65 ml n-Butanol abs. wurde für weitere 15 min gerührt. Hydrolyse erfolgte anschließend durch Zutropfen von 1,09 ml 12,5 n HCl. Die Lösung wurde für 3 h unter Ar weitergerührt und dann lose abgedeckt bis zur vollständigen Gelierung an der Luft stehen gelassen.

Beispiel 16

Amorphes, mikroporöses Zinndioxid-Zirkondioxid-Methylsiliciumsesquioxid-Glas

10,25 ml Zr (OC$_3$H$_7$$^n$)$_4$ (70% ige Lösung in Propanol) und XX ml MeSi(OEt)$_3$ wurden unter Argon vorgelegt und mit einer Lösung bestehend aus 6.25 ml Isopropanol abs., 0.20 ml HNO$_3$ (14 M) und 1.87 ml Acetylaceton versetzt. Die Lösung wurde für 15 min gerührt. Anschließend erfolgte die Zugabe von 0,646 g Sn(2-ethylhexanoat )$_2$. Nach weiteren 15 min Rühren wurde hydrolisiert. Dazu tropfte man eine Lösung aus 6.25 ml Isopropanol abs., 0.30 ml HNO$_3$ ( 14 M ) und 1.88 ml H$_2$O zu. Die klare Lösung wurde für 5 h unter Ar gerührt und dann lose abgedeckt bis zur vollständigen Gelierung an der Luft stehen gelassen.

Beispiel 17

Amorphes, mikroporöses Chromoxid-Aluminiumoxid-Methylsiliciumsesquioxid-Glas

10.00 g Al(Os-Bu)$_3$, XX ml MeSi(OEt)$_3$ und 0.458 g Cr(Oi-Pr)$_3$ wird unter Ar vermischt. Nach 15 min Rühren werden 8,16 ml Isopropanol zugegeben. Anschließend wird durch Zutropfen von 4,40 ml Acetylaceton komplexiert. Dabei tritt eine starke Wärmeentwicklung auf. Die Lösung verfärbt sich gelblich. Nach Säurezugabe (3,04 ml HNO$_3$ (14 M)) und 15 min Rühren erfolgt die Hydrolyse mit einer Lösung aus 8,16 ml Isopropanl und 1,53 ml Wasser. Die klare bräunliche Lösung wird für 2 h unter Ar gerührt und dann lose abgedeckt bis zur vollständigen Gelierung an der Luft stehen gelassen.

Beispiel 18

Epoxidierung von 1-Octen mit tert.-Butylhydroperoxid (TBHP)

15,0 mmol 1-Octen, 1 ml TBHP-Lösung (3m in Isooctan) und 50 mg Titandioxid-Silicium-dioxid-Methylsiliciumsesquioxid-Glas (1 TiO$_2$ : 54,45 SiO$_2$ : 44,55 MeSiO$_{1.5}$) wurden bei 80 C 24 h gerührt und die Produktzusammensetzung gaschromatographisch ermittelt. Bei vollständigem Umsatz betrug die Epoxidselektivität 96%.

Beispiel 19

Epoxidierung von 1-Octen mit tert.-Butylhydroperoxid (TBHP)

15,0 mmol 1-Octen, 1 ml TBHP-Lösung (3m in Isooctan) und 50 mg Vanadiumoxid-Siliciumdioxid-Methylsiliciumsesquioxid-Glas (2,5 V$_2$O$_5$ : 66,5 SiO$_2$ : 28,5 MeSiO$_{1.5}$) wurden bei 80 C 20 h gerührt und die Produktzusammensetzung gaschromatographisch ermittelt. Bei einem Umsatz von 68% wurde eine Epoxidselektivität von 74% erreicht.

Beispiel 20

Monoepoxidierung von t,t,t-1,5,9-Cyclododecatrien (all-trans CDT) mit tert.-Butylhydroperoxid (TBHP)

15,0 mmol all-trans CDT, 1 ml TBHP-Lösung (3m in Isooctan), 2 ml Isooctan und 50 mg Titandioxid-Siliciumdioxid-Methyisiliciumsesquioxid-Glas (1 TiO$_2$ : 48,5 SiO$_2$ : 48,5 MeSiO$_{1.5}$) wurden bei 80 C 24 h gerührt und die Produktzusammensetzung gaschromatographisch ermittelt. Bei einem Umsatz von 88% betrug die Monoepoxidselektivität 89,3%.

Beispiel 21

Epoxidierung von Propen mit tert.-Butylhydroperoxid (TBHP)

150 mg mikroporöser amorpher Ti-Si-oxid-Katalysator (4,8 % Ti) wurden in einen 200 ml Stahlautoklaven gegeben und in 10 ml TBHP-Lösung (3-molar in Isooctan) suspendiert. Anschließend wurde der Autoklav verschlossen und 3,2 g Propen aufgedrückt. Die Reaktion wurde unter Rühren (Rührfisch) bei einer Temperatur von 60°C für eine Dauer von 3h durchgeführt; danach wurde der Reaktor abgekühlt, der Katalysator abzentrifugiert und die Zusammensetzung der klaren Reaktionslösung gaschromatographisch analysiert. Es ergab sich folgende Produktzusammensetzung: 0,4 % Propen, 5,0 % Propylenoxid.

Beispiel 22

Propenoxidation mit einem hydrophoben Ti-Si-Glas

75 mg Katalysator (Titandioxid-Siliciumdioxid-Methylsiliciumsesquioxid-Glas (1 $TiO_2$ : 49,5 $SiO_2$ : 49,5 $MeSiO_{1,5}$)) wurden in einen 100 ml Stahlautoklaven gegeben und in 5 ml TBHP-Lösung (3-molar in Isooctan) suspendiert. Anschließend wurde der Autoklav verschlossen und 1,6 g Propen aufgedrückt. Die Reaktion wurde unter Rühren (Rührfisch) bei einer Temperatur von 60°C für eine Dauer von 3h durchgeführt; danach wurde der Reaktor abgekühlt, der Katalysator abzentrifugiert und die Zusammensetzung der klaren Reaktionslösung gaschromatographisch untersucht. Folgende Produktzusammensetzung wurde gefunden: 0,8 % Propen, 10,3 % Propylenoxid. Die Ausbeute hat sich im Vergleich zum hydrophilen Ti-Si-Glas verdoppelt.

Beispiel 23

Selektivoxidation von Ethanol zu Acetaldehyd mit wässriger Wasserstoffperoxidlösung

21 mmol Ethanol, 7 mmol $H_2O_2$ (25%ig in Wasser) und 50 mg Titandioxid-Siliciumdioxid-Methylsiliciumsesquioxid-Glas (1 $TiO_2$ : 54,45 $SiO_2$ : 44,55 $MeSiO_{1,5}$) wurden bei 45 C 18h gerührt und die Produktzusammensetzung gaschromatographisch ermittelt. Es wurde ein Ethanolumsatz von 14,8% bei einer Acetaldehydselektivität von 91,8% erreicht.

Beispiel 24

Epoxidierung von Cycohexen mit wässriger Wasserstoffperoxidlösung

2 mmol Cyclohexen, 4 mmol $H_2O_2$ (25%ig in Wasser) und 20 mg Titandioxid-Siliciumdioxid-Methylsiliciumsesquioxid-Glas (1 $TiO_2$: 67,9 $SiO_2$ : 29,1 $MeSiO_{1,5}$) wurden bei 50 C 15h gerührt und die Produktzusammensetzung gaschromatographisch ermittelt. Es wurde ein Cyclohexenumsatz von 24,0 % bei einer Epoxidselektivität von 57,8 % ermittelt.

Beispiel 25

Epoxidierung von all-trans-Cyclododecatrien mit wässriger Wasserstoffperoxidlösung

2 mmol all-trans-Cyclododecatrien, 4 mmol $H_2O_2$ (25%ig in Wasser) und 20 mg Titandioxid-Siliciumdioxid-Methylsiliciumsesquioxid-Glas (1 $TiO_2$ : 64,3 $SiO_2$ : 34,7 $MeSiO_{1,5}$) wurden bei 50 C 15h gerührt und die Produktzusammensetzung gaschromatographisch ermittelt. Es wurde ein Cyclohexenumsatz von 24,0 % bei einer Epoxidselektivität von 57,8 % ermittelt.

Beispiel 26

Ammoxidation von Cyclohexanon zum Cyclohexanonoxim mit wässriger Wasserstoff-peroxidlösung und wässriger Ammoniaklösung

5 mmol Cyclohexanon, 7,5 mmol Ammoniak (25%ig in Wasser) und 50 mg Titandioxid-Siliciumdioxid-Methylsiliciumsesquioxid-Glas (1 $TiO_2$ : 54,45 $SiO_2$ : 44,55 $MeSiO_{1,5}$) wurden bei 60 C gerührt und bei dieser Temperatur binnen 4h mit 5 mmol $H_2O_2$ (25%ig in Wasser) versetzt. Nach weiteren 2h bei 60 C hat eine 31 %ige Cyclohexanonumsetzung (gaschromato-graphisch) stattgefunden. Die Cyclohexanonoximselektivität wurde zu 9,1% berechnet.

Beispiel 27

Selektivoxidation von Toluol mit wässriger Wasserstoffperoxidlösung

13 mmol Toluol, 4,5 mmol $H_2O_2$ (25%ig in Wasser), 3,1 ml Acetonitril und 25 mg Titandioxid-Siliciumdioxid-Methylsiliciumsesquioxid-Glas (1 $TiO_2$ : 54,45 $SiO_2$ : 44,55 $MeSiO_{1.5}$) wurden bei 80 C 18h gerührt und die Produktzusammensetzung gaschromato-graphisch ermittelt. Es wurde ein Toluolumsatz von 4,3% bei einer Produktselektivität (Benzaldehyd + Benzylthydroperoxid) von 90,5% erreicht.

Beispiel 28

Selektivoxidation von Ethylbenzol mit wässriger Wasserstoffperoxidlösung

13 mmol Ethylbenzol, 4,5 mmol $H_2O_2$ (25%ig in Wasser), 3,1 ml Acetonitril und 25 mg Titandioxid-Siliciumdioxid-Methylsiliciumsesquioxid-Glas (1 $TiO_2$ : 60 $SiO_2$ : 39 $MeSiO_{1.5}$) wurden bei 80 C 18h gerührt und die Produktzusammensetzung gaschromato-graphisch ermittelt. Es wurde ein Ethylbenzolumsatz von 6,8% bei Produktselektivität von $S_{Acetophenon}$ von 57% bzw. $S_2$-phenylethanol von 14,9% erreicht.

Beispiel 29

Hydroxylierung von Benzol zu Phenol mit wässriger Wasserstoffperoxidlösung

3,36 mmol Benzol, 6,6 mmol $H_2O_2$ (25%ig in Wasser), 5 ml Aceton und 35 mg Vanadiumoxid-Siliciumdioxid-Methylsiliciumsesquioxid-Glas (2,5 $V_2O_5$ : 76 $SiO_2$ : 19 $MeSiO_{1.5}$) wurden bei 60 C 4h gerührt und die Produktzusammensetzung gaschromatographisch ermittelt. Es wurde ein Benzolumsatz von 2,6% bei einer Phenolselektivität von 91% erreicht.

Beispiel 30

Veretherung zur Bildung von tert.-Butylethern

In einem 200ml-Autoklaven wurden 300 mmol Isobuten, 50 mmol 1-Hexanol, 50 mmol 1-Naphthol und 300 mg Zinnoxid-Siliciumdioxid-Methyisiliciumsesquioxid-Glas der Zusammen-setzung 3 $SnO_2$ : 97 $SiO_2$ : 0 $MeSiO_{1.5}$ bei einem Druck von 40 bar $N_2$ und einer Temperatur von 140 C 16h gerührt. Durch gaschromatographische Analyse des Produktgemisches ergab sich eine 1-Naphthyltert.-Butylether-Ausbeute von 39,5% (bezogen auf 1-Naphthol) gegenüber einer 1-Hexyl-tert.-Butylether-Ausbeute von 63,6% (bezogen auf 1-Hexanol). Analog werden bei Verwendung des Katalysators 1,5 $Al_2O_3$ : 97 $SiO_2$ : 0 $MeSiO_{1.5}$ eine 1-Naphthyl-tert.-Butylether-Ausbeute von 67,6 und eine 1-Hexyl-tert.-Butylether-Ausbeute von 37,4% erzielt.
Bei Verwendung des Katalysators 3 $ZrO_2$ : 97 $SiO_2$ : 0 $MeSiO_{1.5}$ wird eine 1-Naphthyl-tert.-Butylether-Ausbeute von 24,4 und eine 1-Hexyl-tert.-Butylether-Ausbeute von 46,1% erzielt.
Darüber hinaus werden zu größeren Anteilen Isobuten- Dimere bzw. -Trimere gebildet.

Beispiel 31

Veretherung zur Bildung von tert.-Butylethern

Analag zu der in Beispiel 20 beschriebenen Vorgehensweise wurden mit einem Zinnoxid-Siliciumdioxid-Methylsiliciumsesquioxid der Zusammensetzung 3 $SnO_2$ : 67,9 $SiO_2$ : 29,1 $MeSiO_{1.5}$ Ausbeuten an 1-Naphthyl-tert.-Butylether bzw. 1-Hexyl-tert.-Butylether von 4,6 bzw. 17,2% erhalten. Analog werden bei Verwendung des Katalysators 1,5 $Al_2O_3$ : 87,3 $SiO_2$ : 9,7 $MeSiO_{1.5}$ eine 1-Naphthyltert.-Butylether-Ausbeute von 43,5 und eine 1-Hexyl-tert.-Butylether-Ausbeute von 56,6% erzielt.
Bei Verwendung des Katalysators 3 $ZrO_2$ : 87,3 $SiO_2$ : 9,7 $MeSiO_{1.5}$ wird eine 1-Naphthyl-tert.-Butylether-Ausbeute von 28,4 und eine 1-Hexyl-tert.-Butylether-Ausbeute von 33,4% erzielt.
Die Reaktionen verlaufen ohne Bildung von Isobuten-Oligomeren.

Beispiel 32

Alkylierung von Aromaten

75 mg Aluminiumoxid-Siliciumoxid-Methylsiliclumsesquioxid (3 $Al_2O_3$, 10 $MeSiO_{1,5}$, 87 $SiO_2$), verdünnt mit der gleichen Menge Quarzsand, werden im Strömungsrohr bei 350 °C im Sauerstoffstrom ausgeheizt. Ein flüssiges Toluol-Ethanol 1:3 Gemisch wird kontinuierlich mit 1 ml/h zum $N_2$-Inertgasstrom (Strömungsgeschwindigkeit 4 ml/min) zudosiert und damit bei 300 °C über das Katalysatorbett geleitet. Der Produktgasstrom zeigt quantitativen Umsatz zu Ethyltoluolen.

Beispiel 33

Propylenoxid-Bildung mit Wasserstoffperoxid

1,9 ml 25%iges $H_2O_2$, 1,6 ml Propen (fl), 75 mg Titanoxid-Siliciumdioxid-Methylsiliciumsesquioxid (1 $TiO_2$, 45 $MeSiO_{1,5}$, 54 $SiO_2$) und 3,1 ml Methanol wurden im verschlossenen Autoklaven 3h auf 60 °C erhitzt. Im Produkt war außer Propenoxid nichts nachweisbar. Im Parallelversuch mit methylgruppenfreiem Al-Si-Glas unter vergleichbaren Bedingungen war kein PO nachweisbar.

**Patentansprüche**

1. Amorphe, mikroporöse Mischoxide mit Mikroporen im Bereich <3 nm Durchmesser und einer Gesamtoberfläche zwischen 20 und 1000 $m^2$/g, **dadurch gekennzeichnet, daß** sie in getrockneter Form eine enge Porenverteilung mit einer Halbwertsbreite <± 10% des Porendurchmessers aufweisen und einen Anteil von 0.1-20 Gew.-% an nicht hydrolysierbaren organischen Gruppen enthalten.

2. Amorphe mikroporöse Mischoxide nach Anspruch 1, **dadurch gekennzeichnet, daß** mindestens 50 % der Mischoxide aus einem oder einem Gemisch der Oxide von Titan, Silicium, Aluminium oder Zirkon besteht.

3. Amorphe mikroporöse Mischoxide nach Anspruch 1, **dadurch gekennzeichnet, daß** mindestens 50 % des Mischoxids aus einem oder einem Gemisch der Oxide von Titan, Silicium, Aluminium oder Zirkon besteht und bis zu 50 Gew.-% aus einem oder mehreren Metalloxiden in atomarer Verteilung aus der Gruppe der Elemente Mo, Sn, Zn, V, Mn, Fe, Co, Ni, As, Pb, Bi, Ru, Re, Cr, W, Nb, Hf, La, Ce, Gd, Ga, In Tl, Ag, Cu, Li, K, Na, Be, Mg, Ca, Sr und Ba besteht.

4. Mikroporöse Mischoxide nach den Ansprüchen 1-3, die zusätzlich bis zu 5 Gew.-% wenigstens eines der Elemente Pt, Rh, Ir, Os, Ru, Re, Ag, Au, Cu, Ni, Pd, Co in hochdisperser Form in metallischem oder nichtmetallischem Zustand enthalten.

**Claims**

1. Amorphous microporous mixed oxides, **characterized by** having, in dried form, a narrow pore size distribution with a half width < ± 10% of the pore diameter of micropores with diameters in the range of < 3 nm and a total surface area of between 20 and 1000 $m^2$/g and containing a fraction of from 0.1 to 20% by weight of non-hydrolyzable organic groups.

2. The amorphous microporous mixed oxides according to claim 1, **characterized in that** at least 50% of the mixed oxide matrix consists of one or a mixture of oxides of titanium, silicon, aluminum or zirconium.

3. The amorphous microporous mixed oxides according to claim 1, **characterized in that** at least 50% of the mixed oxide matrix consists of one or a mixture of oxides of titanium, silicon, aluminum or zirconium, and up to 50% by weight consists of one or more metal oxides in atomic distribution of the group of elements consisting of Mo, Sn, Zn, V, Mn, Fe, Co, Ni, As, Pb, Bi, Ru, Re, Cr, W, Nb, Hf, La, Ce, Gd, Ga, In, Tl, Ag, Cu, Li, K, Na, Be, Mg, Ca, Sr and Ba.

4. The amorphous microporous mixed oxides according to claims 1 to 3, additionally containing up to 5% by weight of at least one of the precious metals Pt, Rh, Ir, Os, Ru, Re, Ag, Au, Cu, Ni, Pd, Co in highly dispersed form in a metallic or non-metallic state.

**Revendications**

1.  Oxydes mixtes microporeux amorphes comportant des micropores ayant un diamètre < 3 nm et une aire totale comprise entre 20 et 1000 $m^2$/g, **caractérisés en ce que**, sous forme séchée, ils ont une étroite distribution des pores, avec une largeur à mi-hauteur < ± 10 % du diamètre des pores, et qui contiennent de 0,1 à 20 % en poids de groupes organiques non hydrolysables.

2.  Oxydes mixtes microporeux amorphes selon la revendication 1, **caractérisé en ce qu'**au moins 50 % des oxydes mixtes sont constitués d'oxydes de titane, de silicium, d'aluminium ou de zirconium, ou d'un mélange de ces oxydes.

3.  Oxydes mixtes microporeux amorphes selon la revendication 1, **caractérisés en ce qu'**au moins 50 % des oxydes mixtes sont constitués d'oxydes de titane, de silicium, d'aluminium ou de zirconium, ou d'un mélange de ces oxydes, et jusqu'à 50 % en poids sont constitués d'un ou plusieurs oxydes métalliques, selon une distribution en atomes, choisis dans l'ensemble comprenant les éléments Mo, Sn, Zn, V, Mn, Fe, Co, Ni, As, Pb, Bi, Ru, Re, Cr, W, Nb, Hf, La, Ce, Gd, Ga, In, Tl, Ag, Cu, Li, K, Na, Be, Mg, Ca, Sr et Ba.

4.  Oxydes mixtes microporeux amorphes selon les revendications 1 à 3, qui contiennent en outre jusqu'à 5 % en poids d'au moins l'un des éléments Pt, Rh, Ir, Os, Ru, Re, Ag, Au, Cu, Ni, Pd, Co sous une forme hautement dispersée à l'état métallique ou non métallique.

Abbildung 1: Abhängigkeit der Ausbeute der Epoxidierung von 1-Octen mit TBHP vom Methylgruppengehalt X amorpher, mikroporöser Titandioxid-Silicium-dioxid-Methylsiliciumsesquioxid-Gläser (1 $TiO_2$ : 99-X $SiO_2$ : X $MeSiO_{1,5}$).

Abbildung 2: Typische Ar-Adsorptionsisotherme und Mikroporenverteilung eines amorphen Aluminiumoxid-Siliciumoxid-Methylsiliciumsesquioxid-Glases (Ti:Si:MeSi = 1:79:20).

Abbildung 3: Abhängigkeit des Hydrophobizitätsindexes HI vom Methylgruppengehalt amorpher, mikroporöser Titandioxid-Siliciumdioxid-Methylsiliciumsesquioxid-Gläser (1 $TiO_2$ : 99-X $SiO_2$ : X $MeSiO_{1,5}$). Der unter identischen Bedingungen gemessene HI von TS-1 ist oben als Referenz eingetragen.